# EUROPÄISCHE PATENTSCHRIFT

(11) **EP 0 547 411 B1**
(45) Veröffentlichungstag und Bekanntmachung des Hinweises auf die Patenterteilung: **03.02.1999**
(21) Anmeldenummer: 92120114.1
(22) Anmeldetag: 25.11.1992
(51) Int. Cl.: C07D 239/52, C07D 239/42

(54) **Verfahren zur Herstellung von 2-substituierten 4,6-Dialkoxypyrimidinen**
Process for the preparation of 2-substituted 4,6-dialkoxypyrimidines
Procédé de préparation de 4,6-dialkoxypyrimidines

(30) Priorität: 26.11.1991 CH 3454/91
(43) Veröffentlichungstag der Anmeldung: 23.06.1993
(73) Patentinhaber: LONZA A.G., CH-3945 Gampel/Wallis (CH)
(72) Erfinder: Escher, André, Dr., Naters (Kanton Wallis) (CH); Previdoli, Felix, Dr., Brig (Kanton Wallis) (CH)
(74) Vertreter: Weinhold, Peter, Dr.

(56) Entgegenhaltungen:
- EP-A- 0 024 200
- EP-A- 0 271 833
- EP-A- 0 400 741
- CH-A- 475 260
- PATENT ABSTRACTS OF JAPAN vol. 013, no. 190 (C-593)1989
- PATENT ABSTRACTS OF JAPAN vol. 016, no. 029 (C-0904)1991
- J. CHEM. SOC., vol.22, no., 1966, page 2031 - 2038, J.A.BEE & F.L.ROSE 'S-TRIAZOLOPYRIMIDINES. PART IV. SYNTHESIS AS POTENTIAL THERAPEUTIC AGENTS'

## Beschreibung

Die Erfindung betrifft ein neues Verfahren zur Herstellung von 2-substituierten 4,6-Dialkoxypyrimidinen der allgemeinen Formel 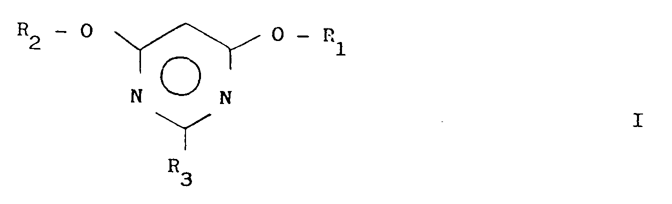 worin R₁ und R₂ gleich oder verschieden sind und eine C₁-C₄-Alkylgruppe bedeuten und R₃ eine R₄-O-, R₄-S- oder 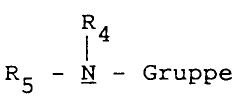 bedeutet, worin R₄ eine C₁-C₄-Alkylgruppe und R₅ ein Wasserstoffatom, eine C₁-C₄-Alkylgruppe oder eine Phenylgruppe bedeuten, ausgehend von einem Cyanimidat.

Diese 2-substituierten 4,6-Dialkoxypyrimidine, insbesondere das 4,6-Dimethoxy-2-(methylthio)pyrimdin sind wichtige Zwischenprodukte zur Herstellung von Herbiziden (EP-A 249 708).

Eine bekannte Ausführungsform zur Herstellung eines Halogen-Pyrimidin-Derivats ist von J.A.Bee & F.L.Rose, J.Chem.Soc., C, 1966, S.2031 beschrieben. Bei diesem Verfahren wird das Halogen-Pyrimidin-Derivat 2-Chlor-4,6-dimethoxy-pyrimidin durch Diazotierung von 2-Amino-4,6-dimethoxy-pyrimidin mit Natriumnitrit und anschliessender Hydrolyse mit konzentrierter Salzsäure synthetisiert.

Ein grosser Nachteil dieses Verfahrens liegt darin, dass das 2-Chlor-4,6-dimethoxypyrimidin in sehr schlechter Ausbeute erhalten wird.

Eine bekannte Ausführungsform zur Herstellung von 4,6-Dialkoxy-2-alkylthiopyrimidinen, ausgehend von 4,6-Dihydroxypyrimidinen und organischen Sulfonsäuren, ist in der JP-A-01 040 470 beschrieben.
Auch dieses Verfahren hat den Nachteil, dass die 4,6-Dialkoxy-2-alkylthiopyrimidine in sehr schlechter Ausbeute erhalten werden.

Desweiteren kann 4,6-Dimethoxy-2-(methylthio)-pyrimidin durch Substitution der Halogenatome in 4,6-Dichlor-2-methylthiopyrimidin mittels Alkali-Methylat hergestellt werden.
Hierbei wird zunächst das Edukt 4,6-Dichlor-2-methylthiopyrimidin durch Chlorierung von 2-Methylthiobarbitursäure mit Phosphoroxidtrichlorid hergestellt (J.Org.Chem. 1961, 26, S.792-803). Anschliessend kann das 4,6-Dichlor-2-methylthiopyrimidin nach fachmännisch üblichen Methoden mittels Alkali-Methylat in 4,6-Dimethoxy-2-(methylthio)-pyrimidin überführt werden.
Dieses Verfahren hat den Nachteil, dass dabei grosse Mengen an Phosphat als Abfallprodukt zur Entsorgung anfallen.

Weiterhin beschreiben Pat. Abs. Jap. 013, 190 (1989) sowie die EP-A-0 271 833 die Herstellung von 2-Aminopyrimidinen.

Die schweizerische Patentschrift CH-A-475 260 offenbart ein Verfahren zur Herstellung von substituierten Pyrimidinen, welche in der 3- bzw. 5-Position eine Aminogruppe aufweisen.

Darüber hinaus schildern die EP-A-0 400 741 sowie Pat. Abs. Jap. 016, 029 (1991) Herbizide, welche in der 2-Position eine substituierte Alkoxygruppe tragen.

Aufgabe der Erfindung war, diese Nachteile zu beseitigen und ein einfaches und ökologisches Verfahren mit guten Ausbeuten zur Herstellung von 2-substituierten 4,6-Dialkoxypyrimidinen zur Verfügung zu stellen.

Diese Aufgabe wurde mit dem neuen Verfahren gemäss Patentanspruch 1 gelöst.

Erfindungsgemäss wird das Verfahren derart ausgeführt, dass man in der ersten Stufe ein Cyanimidat der allgemeinen Formel 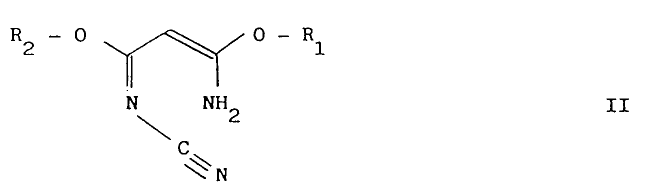 worin R₁ und R₂ die genannte Bedeutung haben, mit einem Halogenwasserstoff zu einem Halogen-Pyrimidin-Derivat der allgemeinen Formel 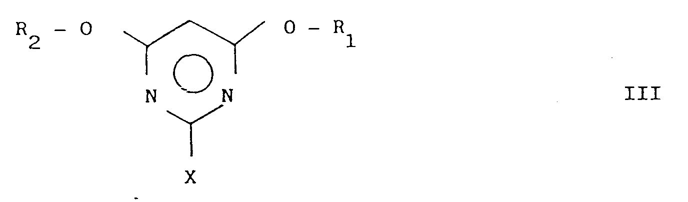 worin R₁ und R₂ die genannte Bedeutung haben und X ein Halogenatom bedeutet, in einem inerten organischen Lösungsmittel cyclisiert, dieses Halogen-Pyrimidin-Derivat dann in der zweiten Stufe entweder mit einer Verbindung der allgemeinen Formel

M - R₃ IV

worin R₃ die genannte R₄-O- oder R₄-S-Gruppe bedeutet und M ein Alkali-Metall-Atom bedeutet oder mit einem Alkylamin der allgemeinen Formel 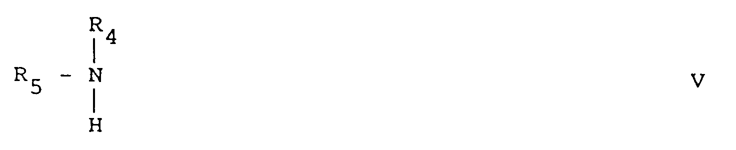 worin R₄ und R₅ die genannte Bedeutung haben, in einem inerten organischen Lösungsmittel in das Endprodukt gemäß Formel I überführt.

Die erste Stufe wird zweckmässig mit Imidaten der allgemeinen Formel 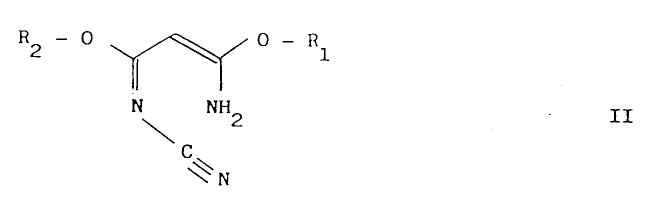 durchgeführt, worin R₁ und R₂ eine Methyl- oder eine Ethylgruppe bedeuten. Vorzugsweise wird als Imidat der Formel II 3-Amino-3-methoxy-N-cyano-2-propenimidat verwendet, worin R₁ und R₂ eine Methylgruppe bedeuten.

3-Amino-3-methoxy-N-cyano-2-propenimidat kann beispielsweise auf einfache Weise gemäss der EP-PS 024 200 hergestellt werden.
Als Halogenwasserstoff können in der ersten Stufe Chlorwasserstoff, Bromwasserstoff oder Jodwasserstoff angewendet werden, vorzugsweise wird Chlorwasserstoff angewendet.

Der Halogenwasserstoff kann in einer Menge von 2 bis 4 mol pro mol Cyanimidat der Formel II angewendet werden, vorzugsweise wird der Halogenwasserstoff dem Reaktionsgemisch gasförmig bis zur Sättigung eingeleitet.

Die Temperaturen bei der Umsetzung in der ersten Stufe liegen zweckmässig zwischen -30 und +30°C, Vorzugsweise zwischen -20 und +10°C.

Als Lösungsmittel können für die Umsetzung in der ersten Stufe beispielsweise Tetrahydrofuran, Toluol, Acetonitril, Methylenchlorid oder niedrig siedende Alkohole angewendet werden, Vorzugsweise Toluol.

Nach einer üblichen Umsetzungsdauer von 1 bis 5 h kann dann das Halogen-Pyrimidin-Derivat der Formel III nach fachmännisch üblicher Methode aufgearbeitet werden, oder direkt, ohne Isolation, für die zweite Stufe eingesetzt werden.

Für die Umsetzung in der zweiten Stufe sind die zweckmässigen Vertreter der Verbindungen der allgemeinen Formel

M - R₃ IV

die, worin R₃ Methanolat oder Ethanolat, vorzugsweise Methanolat, oder Thiolat und M ein Alkalimetallatom, wie beschrieben, bedeuten.
Die vorzugsweisen Vertreter der Formel IV sind: Natrium-, Kaliummethanolat, Natrium- und Kaliumthiolat.

Für die Umsetzung in der zweiten Stufe sind als zweckmässige Vertreter auch Alkylamine der allgemeinen Formel 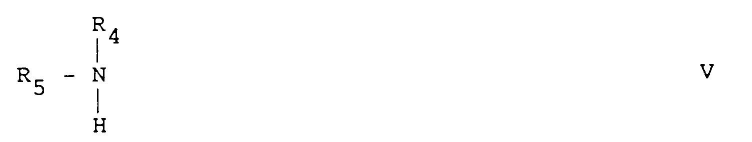 worin R₄ eine C₁-C₄-Alkylgruppe und R₅ eine C₁-C₄-Alkylgruppe oder ein Wasserstoffatom bedeuten, geeignet. Vorzugsweise wird als Alkylamin Butylamin angewendet, worin R₄ eine Butylgruppe und R₅ ein Wasserstoffatom bedeutet.

Die Verbindungen der allgemeinen Formel IV oder V können in einer Menge von 1 bis 3 mol, vorzugsweise von 1 bis 2 mol pro mol Halogen-Pyrimidin-Derivat der Formel III, eingesetzt werden.

Die Temperaturen bei der Umsetzung in der zweiten Stufe liegen zweckmässig zwischen -10 und 100°C, vorzugsweise zwischen 40 und 80°C.
Als Lösungsmittel können für die Umsetzung in der zweiten Stufe die gleichen wie in der ersten Stufe angewendet werden.

Nach einer üblichen Umsetzungsdauer von 1 bis 50 h wird dann das Endprodukt gemäss Formel I nach fachmännisch üblichen Methoden aufgearbeitet.
Vorzugsweise wird die ganze Umsetzung ohne Isolation des Halogen-Pyrimidin-Derivats gemäss Formel III durchgeführt.

Die 2-N-Alkylamino-4,6-dialkoxypyrimidine der allgemeinen Formel 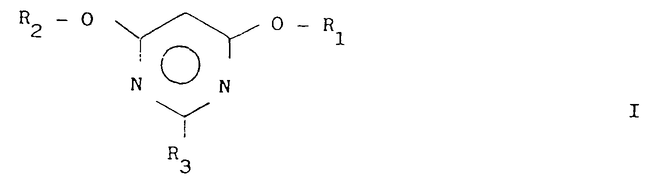 worin R₃ eine 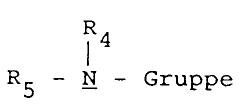 bedeutet, worin R₄ eine C₁-C₄-Alkylgruppe und R₅ ein Wasserstoffatom oder eine C₁-C₄-Alkylgruppe bedeuten, sind neu. Als vorzugsweiser Vertreter dieser neuen Verbindungen wird 2-N-Butylamino-4,6-dimethoxypyrimidin hergestellt.

### Beispiel 1

### Verfahren zur Herstellung von 2-Chlor-4,6-dimethoxypyrimidin

### a) Tetrahydrofuran als Lösungsmittel

4,7 g 3-Amino-3-methoxy-N-cyano-2-prcpenimidat wurden in 60 ml Tetrahydrofuran aufgeschlämmt und auf -20°C abgekühlt. Es wurde Salzsäuregas bis zur Sättigung eingeleitet, wobei die Temperatur im Bereich von -10 bis -20°C gehalten wurde.
Über einen Zeitraum von 3 h wurde in Abständen von 30 min jeweils soviel Salzsäuregas eingeleitet, dass die Lösung wieder gesättigt wurde. Das Tetrahydrofuran wurde vollständig abdestilliert, nach Zugabe von 50 ml Wasser wurde dreimal mit Methylenchlorid extrahiert und die organische Phase nach Trocknung über Natriumsulfat vollständig eingedampft. Man erhielt 4,0 g kristallines weisses Produkt entsprechend einer Ausbeute von 71,1% bezogen auf das eingesetzte Propenimidat.
- Smp:: 99-100°C
- Gehalt:: 94% (GC)
Das Produkt konnte wie folgt umkristallisiert werden: Obiges Rohpyrimidin wurde in 25 ml Isopropanol auf 70°C erwärmt. Nach Wasserzugabe bis zur einsetzenden Trübung liess man auf 10°C abkühlen und filtrierte ab. Nach der Trocknung erhielt man 3,5 g Reinprodukt, was einer Ausbeute von 66,2% bezogen auf das Propenimidat entsprach.
Smp: 102°C
Gehalt: >99% (GC)

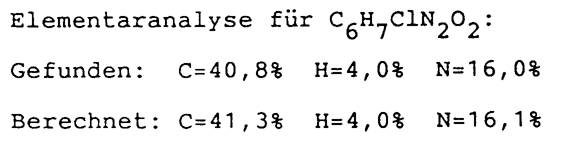

¹H-NMR (CDCl₃, 300 MHz) δ in ppm: 5,97 (s, 1H); 3,95 (s, 6H).

### b) Toluol als Lösungsmittel

Eine Suspension von 2,4 g 3-Amino-3-methoxy-N-cyano-2-propenimidat in 20 ml Toluol wurde bei 0°C mit Salzsäuregas gesättigt. Man rührte die Suspension während 2 h, wobei der HCl-Strom so aufrechterhalten wurde, dass das Reaktionsgemisch immer gesättigt blieb. Es wurde 20 ml Wasser zugegeben, die Phasen getrennt und die Wasserphase noch zweimal mit 10 ml Toluol extrahiert. Die vereinigten organischen Phasen wurde vollständig eingeengt und im Hochvakuum getrocknet. Man erhielt 2,1 g weisses kristallines Produkt mit einem GC-Gehalt von 95%, was einer Ausbeute von 73,9% bezüglich des Propenimidats entsprach. Smp: 100°C

### Beispiel 2

### Verfahren zur Herstellung von 4,6-Dimethoxy-2-methylthiopyrimidin

2-Chlor-4,6-dimethoxypyrimidin wurde wie in Beispiel 1 hergestellt. Die nach Extraktion erhaltenen organischen Phasen wurden bei Raumtemperatur zu einer Lösung von 1,5 Moläquivalenten Natriumthiolat in 5 ml Methanol getropft. Zur Vervollständigung der Reaktion wurde nach 2 h auf 50°C erwärmt und während 2 h bei dieser Temperatur gehalten.
Nach Extraktion mit 20 ml Wasser, Einengen und Trocknen im Hochvakuum erhielt man 1,8 g weisses kristallines Produkt, was einer Ausbeute von 60% bezogen auf das Propenimidat entsprach.

- Smp:: 49-50°C
Durch Umkristallisation aus Isopropanol/Wasser erhielt man ein Produkt mit einem Smp.von 54-56°C.
- ¹H-NMR: (CDCl₃, 300 MHz) δ in ppm: 5,72 (s, 1H); 3,95 (s, 6H), 2,55 (s, 3H)

### Beispiel 3

### Verfahren zur Herstellung von 2-N-Butylamino-4,6-dimethoxypyrimidin

Eine Lösung von 1,9 g 2-Chlor-4,6-dimethoxypyrimidin in 30 ml Toluol (aus Beispiel 1b) wurde mit 2,7 g Butylamin und 3,3 g Triethylamin versetzt und während 50 h bei 80°C gehalten. Nach dem Erkalten wurde zweimal mit 30 ml Wasser extrahiert und dann die organische Phase vollständig eingeengt. Das leicht gelbe Öl wurde bei 140°C/1 mbar destilliert. Man erhielt 2,1 g eines farblosen Öls entsprechend einer Ausbeute von 66% bezogen auf das Propenimidat.
Gehalt: 98% (GC)
¹H-NMR: (CDCl₃, 300 MHz) δ in ppm 5,4 (s, 1H); 4,95 (b, 1H), 3,85 (s, 6H); 3,4 (q, 2H); 1,55 (m, 2H); 1,4 (m, 2H); 0,95 (t, 3H).

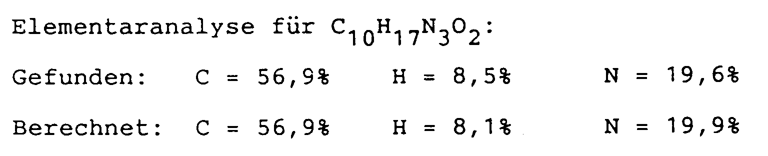

### Beispiel 4

### Verfahren zur Herstellung von 2,4,6-Trimethoxypyrimidin

1,3 g 2-Chlor-4,6-dimethoxypyrimidin aus Beispiel 1a (Gehalt 94%) wurden in 9 ml Methanol gelöst. Nach Zugabe von 2,5 g Natriummethanolatlösung (30%-ig in Methanol) wurde während zwei Stunden bei 55°C gerührt. Nach dem Erkalten wurde das ausgefallene Natriumchlorid abfiltriert und das Filtrat mit 15 ml Wasser versetzt. Beim Stehenlassen im Eisbad kristallisierte das Produkt als feine weisse Nadeln, die abfiltriert und bei Raumtemperatur am Vakuum getrocknet wurden. Man erhielt 1,02 g reines Produkt entsprechend einer Ausbeute von 86%.
- Smp.:: 55°C
- ¹H-NMR:: (CDCl₃, 300 MHz) δ in ppm 5,7 (s, 1H); 4,0 (s, 3H); 3,95 (s, 6H).

## Patentansprüche

1. Verfahren zur Herstellung von 2-substituierten 4,6-Dialkoxypyrimidinen der allgemeinen Formel 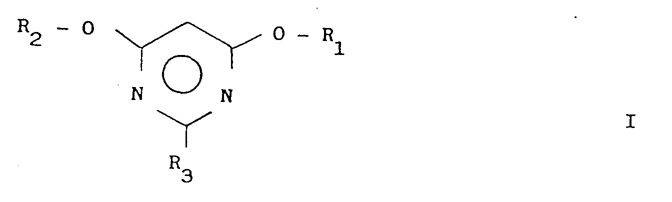 worin R₁ und R₂ gleich oder verschieden sind und eine C₁-C₄-Alkylgruppe bedeuten und R₃ eine R₄-O-, R₄-S- oder 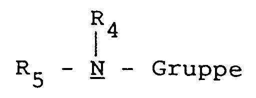 bedeutet, worin R₄ eine C₁-C₄-Alkylgruppe und R₅ ein Wasserstoffatom, eine C₁-C₄-Alkylgruppe oder eine Phenylgruppe bedeuten, dadurch gekennzeichnet, dass man in der ersten Stufe ein Cyanimidat der allgemeinen Formel 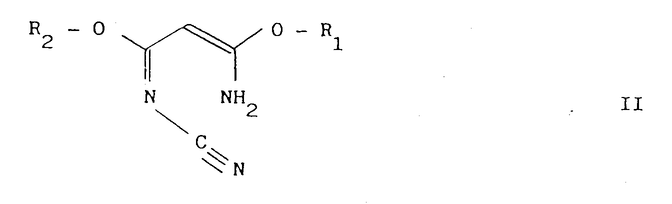 worin R₁ und R₂ die genannte Bedeutung haben, mit einem Halogenwasserstoff zu einem Halogen-Pyrimidin-Derivat der allgemeinen Formel 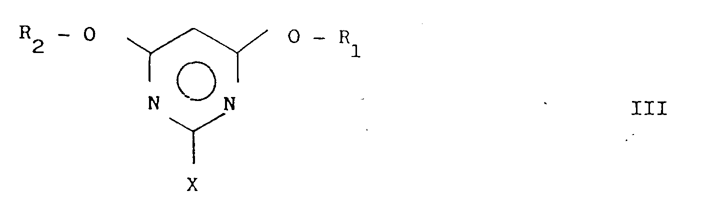 worin R₁ und R₂ die genannte Bedeutung haben und X ein Halogenatom bedeutet, in einem inerten organischen Lösungsmittel cyclisiert, dieses dann in der zweiten Stufe entweder mit einer Verbindung der allgemeinen Formel
M - R₃ IV
worin R₃ die genannte R₄-O- oder R₄-S-Gruppe bedeutet und M ein Alkali-Metall-Atom bedeutet oder mit einem Alkylamin der allgemeinen Formel 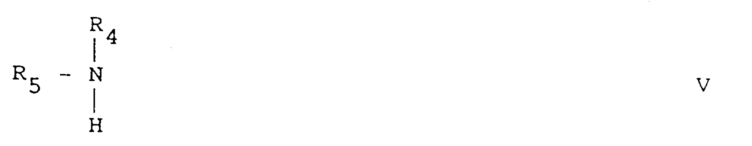 worin R₄ und R₅ die genannte Bedeutung haben, in einem inerten organischen Lösungsmittel in das Endprodukt gemäß Formel I überführt.

2. Verfahren nach Patentanspruch 1, dadurch gekennzeichnet, dass man in der ersten Stufe als Imidat 3-Amino-3-methoxy-N-cyano-2-propenimidat, worin R₁ und R₂ eine Methylgruppe bedeutet, verwendet.

3. Verfahren nach mindestens einem der Patentansprüche 1 und 2, dadurch gekennzeichnet, dass man in der ersten Stufe als Halogenwasserstoff Chlorwasserstoff verwendet.

4. Verfahren nach mindestens einem der Patentansprüche 1 bis 3, dadurch gekennzeichnet, dass man die Umsetzung in der ersten Stufe bei einer Temperatur von -30 bis +30°C durchführt.

5. Verfahren nach einem der Patentansprüche 1 bis 4, dadurch gekennzeichnet, dass man in der zweiten Stufe als Verbindung der allgemeinen Formel IV ein Alkalimetallthiolat oder ein Alkalimetallmethanolat einsetzt.

6. Verfahren nach einem der Patentansprüche 1 bis 4, dadurch gekennzeichnet, dass man in der zweiten Stufe ein Alkylamin der allgemeinen Formel V, worin R₄ eine Butylgruppe und R₅ ein Wasserstoffatom bedeuten, verwendet.

7. Verfahren nach mindestens einem der Patentansprüche 1 bis 6, dadurch gekennzeichnet, dass man die Umsetzung in der zweiten Stufe bei einer Temperatur von -10 bis 100°C durchführt.

8. Verfahren nach mindestens einem der Patentansprüche 1 bis 7, dadurch gekennzeichnet, dass man die Umsetzung ohne Isolation des Halogen-Pyrimidin-Derivats gemäss Formel III durchführt.

9. Verfahren zur Herstellung von Halogen-Pyrimidin-Derivaten der allgemeinen Formel 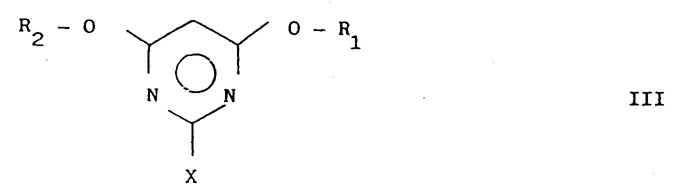 worin R₁,R₂ und X die genannte Bedeutung haben, dadurch gekennzeichnet, dass man ein Cyanimidat der allgemeinen
Formel 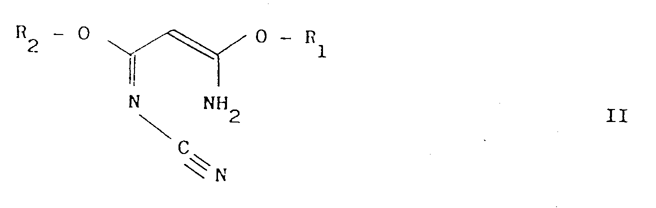 worin R₁ und R₂ die genannte Bedeutung haben, in einem inerten organischen Lösungsmittel mit einem Halogenwasserstoff umsetzt.

## Claims

1. A method for producing 2-substituted 4,6-dialkoxypyrimidines having the general formula 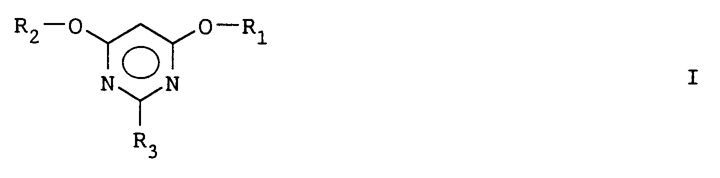 wherein R₁ and R₂ are the same or different and represent a C₁-C₄ alkyl group and R₃ represents an R₄-O-, R₄-S- or 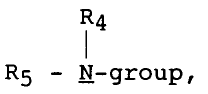 wherein R₄ represents a C₁-C₄ alkyl group and R₅ represents a hydrogen atom, a C₁-C₄ alkyl group or a phenyl group, characterised in that in the first step a cyanoimidate having the general formula 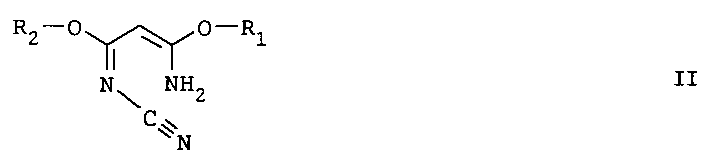 wherein R₁ and R₂ have the above meaning, is cyclised with a hydrogen halide in an inert organic solvent to form a halopyrimidine derivative having the general formula 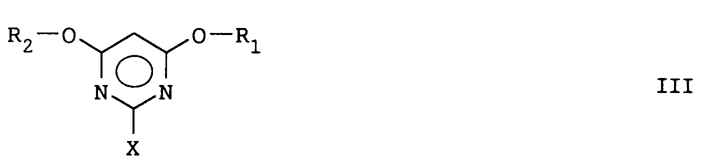 wherein R₁ and R₂ have the above meaning and X represents a halogen atom, which is then, in the second step, reacted in an inert organic solvent either with a compound having the general formula
M ― R₃ IV
wherein R₃ represents the above R₄-O- or R₄-S-group and M represents an alkali metal atom, or with an alkylamine having the general formula 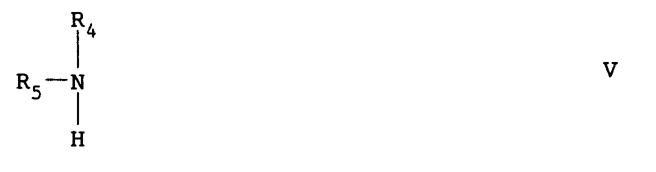 wherein R₄ and R₅ have the above meaning, to form the final product of Formula I.

2. The method according to claim 1, characterised in that 3-amino-3-methoxy-N-cyano-2-propeneimidate, wherein R₁ and R₂ represent a methyl group, is used in the first step as an imidate.

3. The method according to at least one of claims 1 and 2, characterised in that hydrogen chloride is used in the first step as a hydrogen halide.

4. The method according to at least one of claims 1 to 3, characterised in that reaction in the first step is carried out at a temperature of from -30 to +30°C.

5. The method according to any one of claims 1 to 4, characterised in that an alkali metal thiolate or an alkali metal methanolate is employed in the second step as a compound of the general Formula IV.

6. The method according to any one of claims 1 to 4, characterised in that in the second step an alkylamine of the general Formula V is used, wherein R₄ represents a butyl group and R₅ represents a hydrogen atom.

7. The method according to at least one of claims 1 to 6, characterised in that reaction in the second step is carried out at a temperature of from -10 to 100°C.

## Revendications

1. Procédé pour la préparation de 4,6-dialcoxypyrimidines 2-substituées selon la formule générale 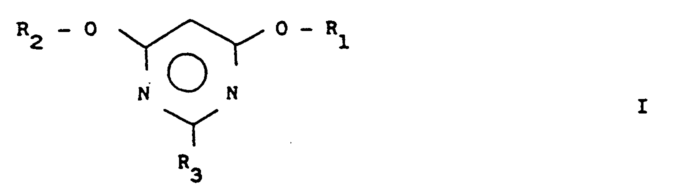 dans laquelle R₁ et R₂ sont identiques ou différents et signifient un groupe alkyle C₁-C₄ et R₃ signifie un groupe R₄-O-, R₄-S- ou 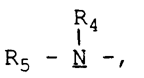 dans laquelle R₄ signifie un groupe alkyle C₁-C₄ et R₅ un atome d'hydrogène, un groupe alkyle C₁-C₄ ou un groupe phényle, caractérisé en ce que dans la première étape, on cyclise dans un solvant organique inerte un cyanimidate de la formule générale 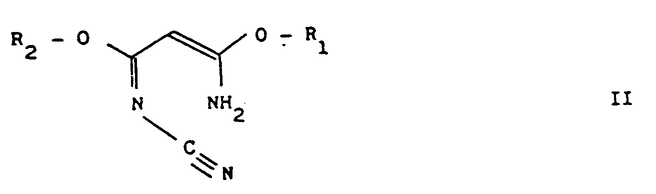 dans laquelle R₁ et R₂ ont la signification citée, avec un halogénohydrogène en un dérivé halogène-pyrimidine de la formule générale 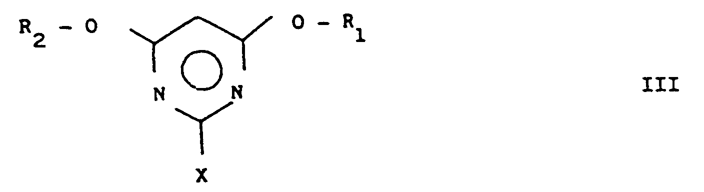 dans laquelle R₁ et R₂ ont la signification citée et X signifie un atome halogène, en ce que l'on transforme ce solvant organique inerte dans la deuxième étape soit avec un composé de la formule générale 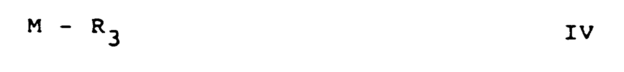 dans laquelle R₃ signifie le groupe R₄-O- ou R₄-S- cité et M signifie un atome de métaux alcalins ou avec une alkylamine de la formule générale 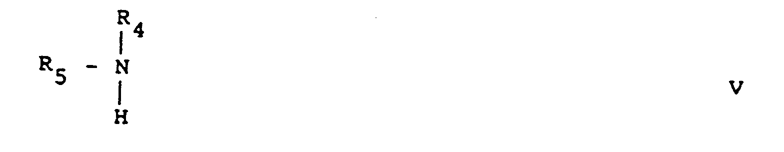 dans laquelle R₄ et R₅ ont la signification citée, dans un solvant organique inerte pour donner le produit final selon la formule I.

2. Procédé selon la revendication 1, caractérisé en ce que dans la première étape en tant qu'imidate, on utilise le 3-amino-3-méthoxy-N-cyano-2-propènimidate, dans lequel R₁ et R₂ signifient un groupe méthyle.

3. Procédé selon au moins l'une des revendications 1 et 2, caractérisé en ce que dans la première étape, on utilise en tant qu'halogénohydrogène, du chlorure d'hydrogène.

4. Procédé selon au moins l'une des revendications 1 à 3, caractérisé en ce que l'on conduit la réaction dans la première étape à une température de -30 à +30°C.

5. Procédé selon l'une des revendications 1 à 4, caractérisé en ce que dans la deuxième étape, on met en oeuvre en tant que composé de la formule générale IV, un thiolat des métaux alcalins ou un méthanolat des métaux alcalins.

6. Procédé selon l'une des revendications 1 à 4, caractérisé en ce que dans la seconde étape, on utilise une alkylamine de la formule générale V dans laquelle R₄ signifie un groupe butyle et R₅ un atome d'hydrogène.

7. Procédé selon au moins l'une des revendications 1 à 6, caractérisé en ce que l'on conduit la réaction dans la deuxième étape à une température de -10 à 100°C.
